(19) Europäisches Patentamt / European Patent Office / Office européen des brevets

(11) **EP 4 679 066 A1**

(12) **EUROPEAN PATENT APPLICATION**

(43) Date of publication:
14.01.2026 Bulletin 2026/03

(21) Application number: 25184712.5

(22) Date of filing: 24.06.2025

(51) International Patent Classification (IPC):
*G01N 23/046* (2018.01)    *A61B 6/03* (2006.01)

(52) Cooperative Patent Classification (CPC):
G01N 23/046; A61B 6/027; A61B 6/03;
A61B 6/032; A61B 6/06; A61B 6/4007;
A61B 6/4014; A61B 6/4028; A61B 6/4085;
G01N 2223/316; G01N 2223/419; G01N 2223/645;
G01N 2223/646

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 09.07.2024 IT 202400015823

(71) Applicant: **Istituto Nazionale di Fisica Nucleare
00044 Frascati (RM) (IT)**

(72) Inventors:
• **SARNO, Antonio
I-00444 FRASCATI (RM) (IT)**
• **CARDARELLI, Paolo
I-00444 FRASCATI (IT)**

• **PATERNÒ, Gianfranco
I-00444 FRASCATI (RM) (IT)**
• **PARDI, Silvio
I-00444 FRASCATI (RM) (IT)**
• **MOLLO, Carlos Maximiliano
I-00444 FRASCATI (RM) (IT)**
• **DE ASMUNDIS, Riccardo
I-00444 FRASCATI (RM) (IT)**
• **PUGLIESE, Mariagabriella
I-00444 FRASCATI (RM) (IT)**
• **MINOPOLI, Antonio
I-00444 FRASCATI (RM) (IT)**

(74) Representative: **Metroconsult Srl
Foro Bonaparte 51
20121 Milano (IT)**

(54) **SCANNER FOR X-RAY CONE-BEAM COMPUTED TOMOGRAPHY (CBCT) BASED ON A MULTI-SOURCE CONFIGURATION FOR QUANTITATIVE EVALUATIONS**

(57)    Described herein is a device for X-ray cone-beam computed tomography, comprising:
- a rotation system (R) for rotating said device around an axis of rotation (D);
- a support system (P) for supporting said device;
- an emission system (T) adapted to emit multiple X-ray beams (S);
- an adjustable collimation system (W) adapted to shape said X-ray beams, said collimation system being placed in front of said emission system (T);
- a detection system (C) for detecting the X-rays emitted by said emission system (T) and collimated by said collimation system (W),
- said emission system (T) and said collimation system (W) being positioned on a first side of said support (P), and said detection system (C) being positioned on a second side of said support (P), opposite the first side;
- a control system (CPU) adapted to acquire projective views of said X-rays detected by said detection system (C) through one or more acquisition cycles, such that:
- the complete scanning of a volume of interest of an object or an anatomical region (E) placed between said collimation system (W) and said detection system (C) is effected through acquisition of projective images during multiple scan cycles, each cycle including the acquisition of projective views from more than one of said multiple sources (S);
- the X-ray beams emitted by said sources (S) are collimated in such a way as to entirely cover the extension of a field of view (FOV) for each scan cycle;
- the entire tomographic acquisition includes more than one scan cycle, carried out by rotating said rotation system (P) around a volume of interest of said object or anatomical region (E).

**FIG. 1**

**Description**

**Field of the invention**

The present invention relates to a scanner for X-ray cone-beam computed tomography (CBCT) based on a multi-source configuration for quantitative evaluations.

**Technical problem to be solved**

Notwithstanding its widespread use, cone-beam tomography (CBCT) has some limitations which are mainly related to two intrinsic features of such technology: 1) the conical shape of the X-ray beam, with the source confined in a circular orbit, and 2) the abundance of scattered radiation that reaches the detector. These limitations undermine the use of CBCT in all those applications that require accuracy, precision, reproducibility and repeatability of quantitative evaluations in reconstructed three-dimensional tomographic images.

As a matter of fact, given the Tuy-Smith condition, in traditional CBCT where source and detector follow a circular orbit the above-mentioned three-dimensional image can only be reconstructed in the plane where the orbit of the source lies. Away from that plane, there is a reduction in the definition of the reconstructed image, i.e. reconstruction approximation, resulting in reduced detail visibility as the distance from the plane increases, along with reduced accuracy of quantitative evaluations. The limitation caused by the wide beam and field of view, as well as by the detector not being able to discriminate between primary X-photons (which have not interacted in the scanned object) and scattered X-photons, generates artifacts in the reconstructed image, such as cup-shaped artifacts introducing in the image a trend in the radial direction (i.e. in the direction perpendicular to the axis of rotation/isocenter around which the source and the detector rotate). Moreover, scattered radiation introduces high-frequency noise in the reconstructed images. Such effects contribute to decreasing the accuracy and, respectively, the precision of the quantitative estimates made in the images. In addition, they reduce the reproducibility and repeatability of such quantitative evaluations. These limitations confine the current CBCT technology to qualitative analyses, preventing or limiting its use in any application requiring quantitative evaluations in the image.

**Description of the prior art**

Cone-beam computed tomography (CBCT) is a tomographic scanning technology that makes use of multiple radiographic projections, taken from different angular positions, of an object, a living being, or an anatomical portion, for the purpose of reconstructing a three-dimensional map of its internal conformation, i.e. a three-dimensional map of the linear attenuation coefficients of the materials contained in the field of view.

The constituent elements of CBCT are, therefore, a source of X-radiation and a digital detector integrally rotating around the object or anatomical portion to be scanned, with the latter always interposed between the source-detector pair. Such integral rotation of the source-detector pair permits the acquisition of several radiographic views of the object or anatomical portion, and, through the use of suitable reconstruction algorithms, it is possible to obtain a three-dimensional distribution representing said map of the attenuation coefficients of the materials within the field of view.

In some configurations, mostly used in industrial environments, the source-detector pair can also move in orbits other than a perfectly circular one confined in a single plane in space. The X-rays generated by the source have a maximum energy on the order of 150 keV for medical applications, and may even reach 360 keV in quality control applications for industrial products. In its simplest configuration, a CBCT scanner includes a motor that produces the integral rotation of the detector and the source around the object or anatomical region to be scanned.

CBCT is widely used for clinical evaluations, particularly because of its flexibility, low cost, and high benefit-to-cost ratio. Its main applications include maxillofacial scanning, image-guided radiotherapy, pre-surgery and post-surgery checks, and limb analyses. It is also widely employed in metrology and for checking industrial products, components and semifinished products.

**Brief description of the invention**

It is therefore the object of the present invention to propose a scanner for X-ray cone-beam computed tomography (CBCT) based on a multi-source configuration for quantitative evaluations, which can overcome all of the above-mentioned drawbacks.

The invention comprises a device for X-ray cone-beam computed tomography, which comprises a plurality of X-ray sources, an adjustable collimation system for shaping the X-ray beams, a detection system, a movement and rotation system, and a control system that performs the acquisition of projective views through acquisition cycles, such that:

- the complete scanning of the volume of interest is effected through acquisition of projective images during multiple scan cycles, each one including the acquisition of projective views from more than one of said sources;
- the X-ray beams emitted by such sources are collimated in such a way as to entirely cover the extension of the field of view (FOV) for each scan cycle;
- the entire tomographic acquisition includes more than one scan cycle, carried out by rotating the source-detector system around the volume of interest.

A software program performs the reconstruction of the tomographic volume.

In an alternative configuration, the plurality of sources are arranged along a segment that is parallel to the axis of rotation.

In an alternative configuration, the detection system is not rotatably integral with the system that comprises the plurality of sources.

In an alternative configuration, said sources are arranged on an arc of circumference and the beams are rotated.

In an alternative configuration, said sources are not coplanar to one another or arranged on the same segment.

In an alternative configuration, the acquisition cycle is carried out by acquiring projective views from a subset of the sources that constitute the system of sources.

In an alternative configuration, the different sources are controlled during the rotation in such a way as to create a quasi-sinusoidal scan orbit.

In an alternative configuration, the sources are controlled in such a way as to delineate a sawtooth (raster) scan orbit.

In an alternative configuration, the present invention is part of a C-arm or a centering system of a radiotherapy linear accelerator (LINAC).

The present invention concerns a device for X-ray cone-beam computed tomography as claimed in claim 1.

The dependent claims concern some preferred variants of the device of claim 1. All claims are intended as integral parts of the present description.

**Brief description of the drawings**

Further objects and advantages of the present invention will become apparent in light of the following detailed description of an exemplary embodiment (and variants thereof) referring to the annexed drawings, which are merely supplied by way of non-limiting example, wherein:

Figure 1 shows a schematic diagram of the scanner of the invention;
Figure 2 shows a graph describing how the invention operates;
Figure 3 shows a schematic plan view (a) and a schematic side view (b) of the scanner;
Figure 4 shows a graph describing an alternative operation of the invention, wherein the sources Si are directly controlled by the CPU;
Figure 5 shows a graph describing an alternative operation of the invention, wherein the sources Si make use of shutters;
Figure 6 shows a diagram of the source assembly coupled with a shutter;
Figure 7 shows an alternative diagram of the scanner of the invention, wherein the detection system C is not rotatably integral with the source system T;
Figure 8 shows an alternative schematic side view of the scanner with a movable detection system;
Figure 9 shows an alternative schematic side view of the scanner, which comprises a plurality of detectors constituting the detection system;
Figure 10 shows a schematic plan view (a) and a schematic side view (b) of a scanner portion comprising a single source;
Figures 11 and 12 show a first and, respectively, a second system comprising the scanner of the invention.

In the drawings, the same reference numerals and letters identify the same items or components.

**Detailed description**

The present invention concerns a scanner for X-ray cone-beam computed tomography with multiple sources for quantitative studies (qCBCT) .

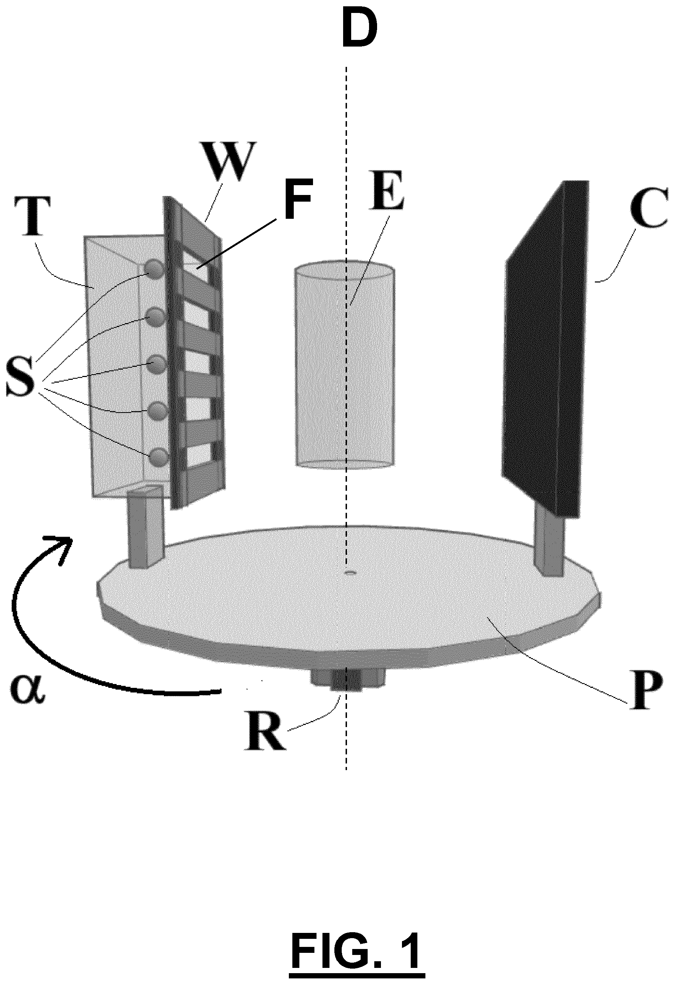

**Figure 1** shows an illustrative diagram of the invention, which depicts the main elements thereof.

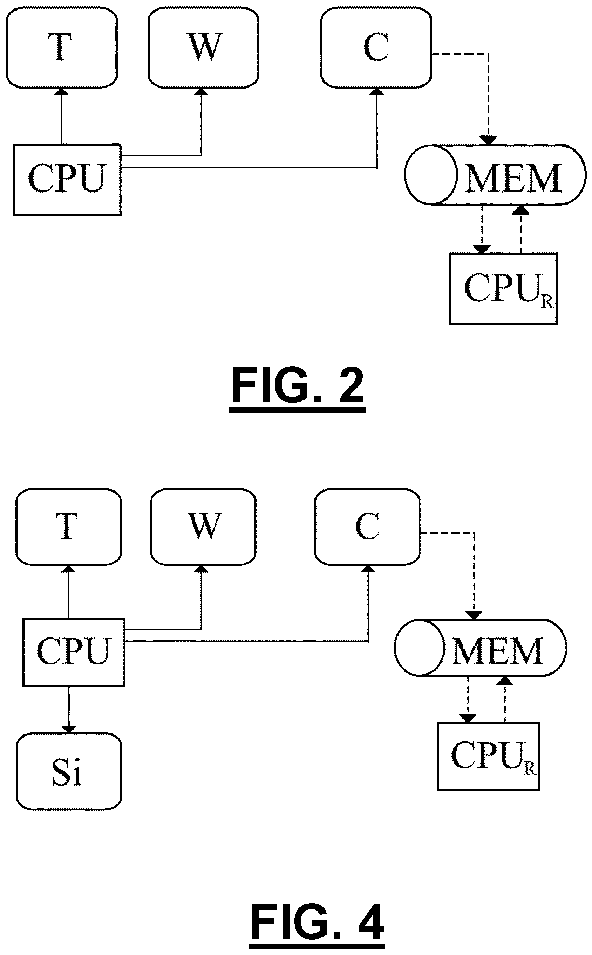

T designates an assembly or system of X-ray sources (S) integral with one another in space, to be understood as focal spots, i.e. the points where X-rays are generated) generated by one or more X-ray tubes or as multiple X-ray sources, each one having one or more focal spots. W designates a system of collimators that model the shape and size of the output X-ray beam. C designates a digital detector or X-ray detection system. P designates a support system whose function is to connect the movements, mainly rotations about an axis, of T, W and C. R designates a system or stage for providing the integral rotation of T or C and R around an axis of rotation D. E designates an object or anatomical region comprised within the field of view of the scanner. ($\alpha$) is the angle of rotation about the axis D. Further constituent elements of the scanner include a control and processing unit CPU, a memory unit MEM, and a second processing unit $CPU_R$, which will be described later on (**Figure 2**).

The basic elements of the qCBCT scanner are, therefore, an assembly or system (T) comprising more than one X-ray source (S), a collimator (W) dedicated to defining the shape and size of the X-ray beam, integral with said assembly or system T, and an X-ray detector (C).

The X-ray sources (S) may be either conventional X-ray tubes assembled into the system T or a multi-spot source, i.e. a single source having multiple focal points or spots from which X-rays are generated. This means that the system T may be an assembly of multiple sources, a multi-spot source, or a single-spot source of X-rays or X-photons.

Such sources may generate equal or different X-ray spectra, i.e. they may operate on equal or different anode voltages or currents. A filter system, not shown in the drawing, at the exit of the X-ray beams may be used for modelling such spectra. The mechanical system R rotatably drives the integral assembly *T, W* and C around an axis of rotation, in proximity to which there is the field of view *FOV* that includes the sample, or anatomical region, or animal, or object to be scanned E. Such rotation is defined by a maximum angle, or scan angle, $\theta$.

The collimator *W* is made of a material with a high atomic number (typically tungsten, lead or tin) or an alloy of materials having a high attenuation coefficient, and only lets the X-ray beam pass through suitable slots F.

Such slots F have variable sizes. Their dimensions can be defined/modified either manually or through electronic stages providing rotations or translations of portions of the collimator.

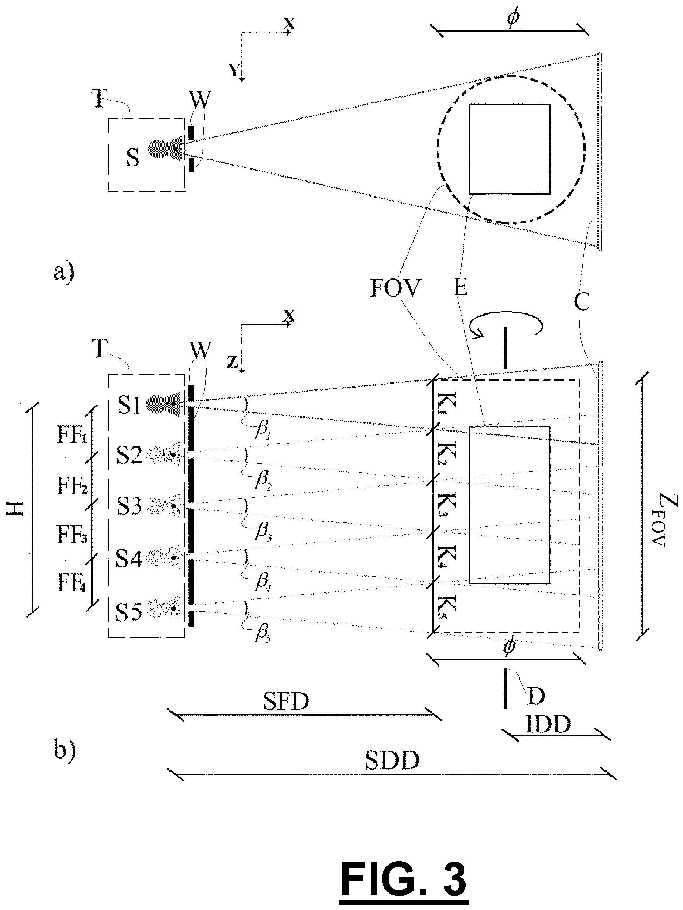

The slots F thus obtained spatially limit the beam to an aperture having a size almost equal to $\phi$ in proximity to the axis of rotation D (**Figures 1 and 3**) in the plane perpendicular to said axis of rotation D, thereby determining the *FOV* diameter in such plane.

More particularly, **Figure 3** shows schematic views of the scanner from above (a) and from a side (b). In this illustrative diagram there are 5 sources $S_i$ (which can be generalized to a plurality N>1) with a source index i such that i = 1... N, each one separated by a distance $FF_i$ with i = 1...N-1. The sum of the distances $FF_i$ for i=1..N-1 is equal to H. The collimation module W limits every single X-ray beam produced, such that in a plan view its aperture is $\phi$ at the isocenter, and such that in a side view it has an angular aperture $\beta_i$. Such lateral aperture determines a linear aperture of the beam on the outer side of the field of view FOV which is equal to $K_i$, such that the sum of all $K_i$ for i=1...N is equal to the length of the FOV in the axial direction $Z_{FOV}$, and that all $K_i$ are contiguous in the axial direction.

In the plane defined by the axis of rotation D and by the individual source $S_i$, the slots F of the collimator W limit the X-ray beam to an angular aperture $\beta_i$ that produces a footprint of the primary beam which is projected onto the lateral surface of the *FOV* at a distance *SFD* from the focal spot equal to $K_i$. The sum of all $K_i$ for i = 1...N is equal to the length of the *FOV* in the direction of the axis of rotation, $Z_{FOV}$ (**Figure 3**).

In an alternative configuration, the footprints $K_i$ have portions overlapping the distance SFD from the focal spot, and their sum has a length greater than $Z_{FOV}$.

During the rotation of *T, W* and *C* around the axis of rotation *D,* the control unit (CPU) coordinates the operation of the detector, rotator and source in order to activate and synchronize them according to the current angular position during the rotation ($\alpha$) (**Figure 2**).

Said control unit CPU makes it possible to acquire a radiographic projection by using one of the N sources $S_i$, suitably selected on the basis of the angular position of *T, W* and *C* during the circular scan.

An acquisition cycle (CA) being defined as a sequence in which projections are acquired from each one of the N sources $S_i$, an entire tomographic scan consists of CA>1. A suitable memory unit MEM is used to store the images acquired by the detector C, and a processing unit $CPU_R$ processes and reconstructs the 3D image starting from the acquired projections (**Figure 2**). MEM can also store additional information, including the positions of the components during the projection. Such information may be obtained from the states of the various components of the scanner. The control unit CPU may also control the movements and operation of the individual sources Si (**Figure 4**), or it may control the assembly or system T, which implicitly operates the sources Si.

As an alternative to the above, once CA has been defined, FOV can be defined as the set of points in 3D space which have at least one projection on the detector per CA. Therefore, said CPU controls, as a function of the value of the angle of rotation $\alpha$, the activation of the i-th source $S_i$ and of the detector *C* in order to acquire the radiographic view.

An alternative scan configuration may use a subset of the sources Si constituting the assembly T, the collimation system W defining apertures of the X-ray beams such that the entire extension of $Z_{FOV}$ is covered.

The scanner may operate in step-and-shoot mode, wherein the integral assembly *T, W* and *C* reaches a certain predefined angle $\alpha$ and then acquires the projection by activating the source $S_i$ for a duration $\Delta t$; as an alternative, it may operate in continuous or pulsed mode, wherein the control unit CPU activates the source $S_i$ for a period $\Delta t$ necessary for

source acquisition when the predefined angle of rotation $\alpha$ is reached, without the rotation being interrupted.

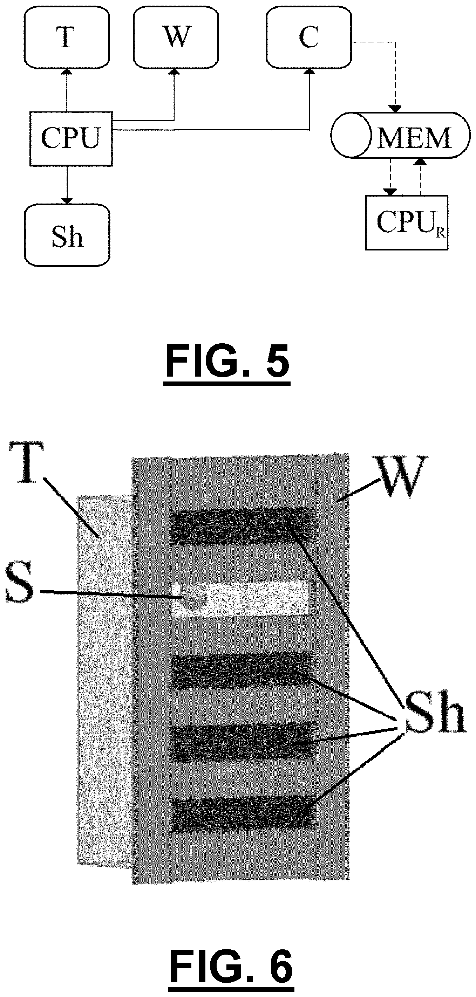

In the same period $\Delta t$, the detector C acquires the radiographic image, which is then digitized and sent to the storage system MEM. As an alternative to activating the source $S_i$, the CPU may command the opening of a shutter Sh (**Figure 5, Figure 6**) for a time $\Delta t$ necessary to acquire the projection, with the source $S_i$ turned on for a period different from $\Delta t$. The control system CPU can also modify the apertures and shapes of the septa of the collimator $W$, both before and during the scan. By way of example, the unit CPU may activate the sources $S_i$ of the schematic diagram shown in **Figure 3b**, i.e. acquire projections according to the following logic, wherein the emission of the X-ray beams follows a quasi-sinusoidal trajectory on a semi-period:

$$(1)\ \left(\frac{\alpha}{\alpha_0} \geq n\ \cap\ \frac{\alpha}{\alpha_0} \leq n + 0.5\right) e\ \frac{H}{2} \cdot \cos\left(\frac{2\pi\alpha}{\alpha_0}\right) = \begin{cases} \frac{H}{2} & projection\ acquired\ from\ S1 \\ \frac{H}{4} & projection\ acquired\ from\ S2 \\ 0 & projection\ acquired\ from\ S3 \\ -\frac{H}{4} & projection\ acquired\ from\ S4 \\ -\frac{H}{2} & projection\ acquired\ from\ S5 \end{cases}$$

where $n$ is an integer smaller than or equal to CA, $\alpha_0$ is a parameter defining the angular period of repetition of said acquisition cycle, and $\theta$ is greater than or equal to $\alpha_0/2$, in order to execute at least one scan cycle CA. This scan logic can be generalized for any number N of sources Si

$$(2)\quad \left(\frac{\alpha}{\alpha_0} \geq n\ \cap\ \frac{\alpha}{\alpha_0} \leq n + 0.5\right) e\ \frac{H}{2} \cdot \cos\left(\frac{2\pi\alpha}{\alpha_0}\right) = \frac{H}{2} \cdot \cos\left(\frac{\pi i}{N}\right)\ projection\ acquired\ from\ Si$$

Subsequent acquisitions in an acquisition cycle CA may also occur at different distances, i.e. they may change from one CA to the next. At any rate, for each CA, all $K_i$ must be *contiguous* or overlapped in the axial direction, and their sum must be greater than or equal to $Z_{FOV}$.

Alternatively, the sources $S_i$ may be activated in a random, but correlated, manner, i.e. not sequentially as a function of the result of the left side of eq. (2).

Considering, by way of example, a co-sinusoidal scan orbit, this may assume any shape, even an irregular one, on condition that the scan cycle CA allows the acquisition of every point of the designated FOV and all $K_i$ are contiguous or overlapped in the axial direction. As a further example, the scan trajectory may follow that of a sawtooth signal (i.e. a periodic signal with a linear pattern within the period).

In this case, therefore, the projection is acquired by using the source $S_i$ in the period $\alpha_0$ as follows:

$$(3)\quad H \cdot \left(1 - \frac{\alpha}{\alpha_0}\right) = \frac{H}{N-1} \cdot (n - 1)\ projection\ acquired\ from\ Si$$

where $\alpha \leq \alpha 0$ for a set period CA. In this example as well, it is possible to not follow a consequential logic in the selection of the sources $S_i$.

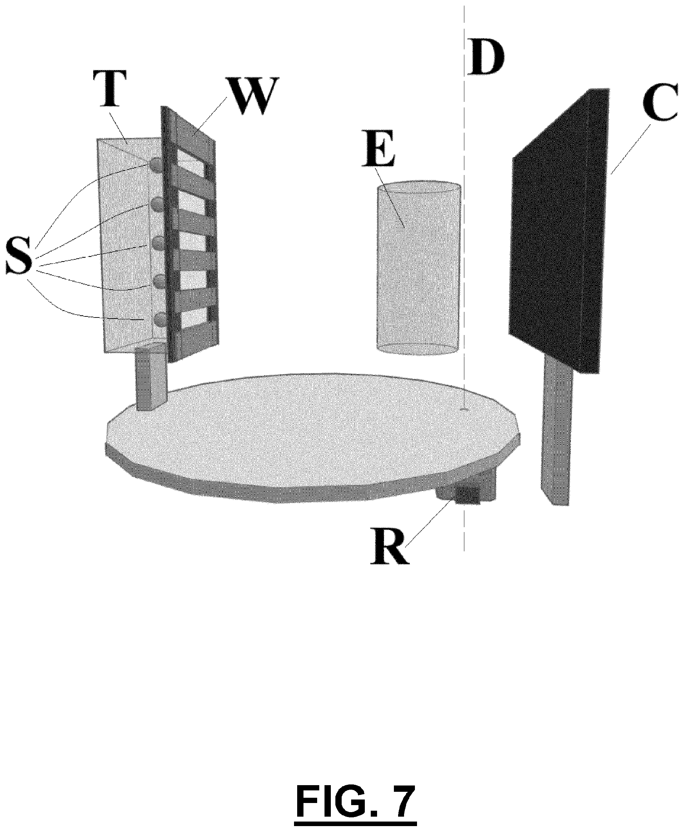

In an alternative configuration, the detector C is fixed during the rotation (tomosynthesis) **(Figure 7)**. In this configuration, the total scan angle is <180 degrees, and the distance between $S_i$ and D (SID = SFD + $\Phi/2$) may be greater than SDD.

For each acquisition cycle it cannot be excluded to associate a different $K_i$ with a given source $S_i$, thus modifying the shape and size of the collimator W during the scan.

Once the radiographic projection has been acquired, an electronic circuit integrated into the detector, or a software program, appropriately limits (by setting suitable windows) the acquired projection so as to eliminate the image portion outside the primary beam produced by the source $S_i$, whose size on the detector is almost equal to $K_i'=K_i \cdot$SDD/SFD in the axial direction, where SDD is the distance between source and detector, and SFD is the distance between the source and the radially external surface of the FOV where $K_i$ is being considered **(Figure 2)**.

In the plane perpendicular to the axis of rotation, this operation determines a footprint of the primary beam on the detector C which is equal to approximately $\phi'=\phi \cdot$SDD/(SDD-IDD), where IDD is the distance between the axis of rotation and the detector **(Figure 2)**.

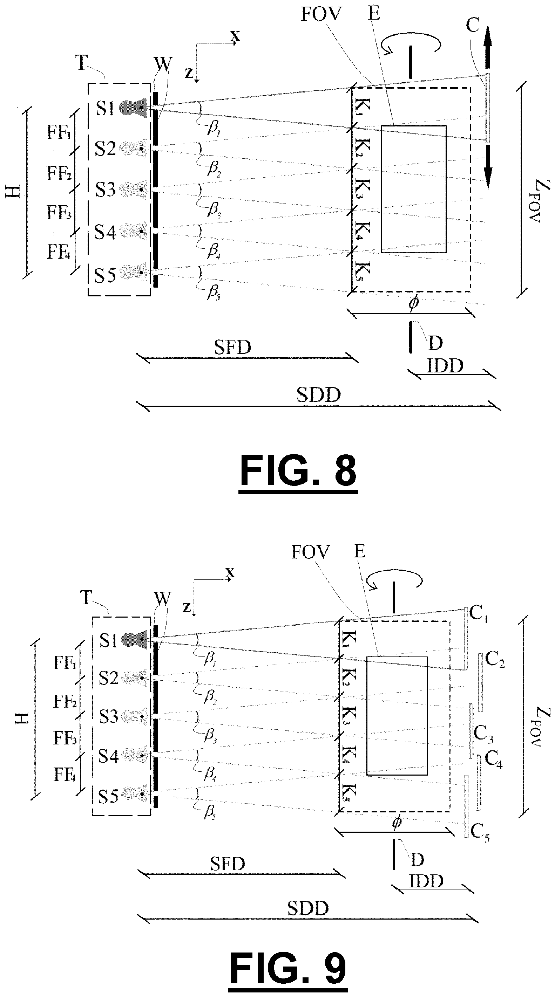

Projection size limitation may also be achieved through the use of a detector C having a size almost equal to $K_i'$ in the axial direction and $\phi'$ in directions perpendicular to the axial one, i.e. having an active area corresponding to such dimensions. In this alternative solution, in order to allow the acquisition of projections from each one of the sources Si, the detector must be translated in the axial direction during the acquisition (**Figure 8**), or multiple detectors (Ci) must be used whose transverse dimensions are such that each one can detect the corresponding source Si (**Figure 9**). If the detectors Ci are smaller than the beam footprint, the projective image of the source $S_i$ can be obtained by joining together (stitching) the images acquired by different detectors. When multiple detectors are used, they may also have different distances IDD from the axis of rotation, i.e. they may not be coplanar to one another. In addition to said windowing, the projective images can be suitably manipulated by means of a computer prior to 3D reconstruction.

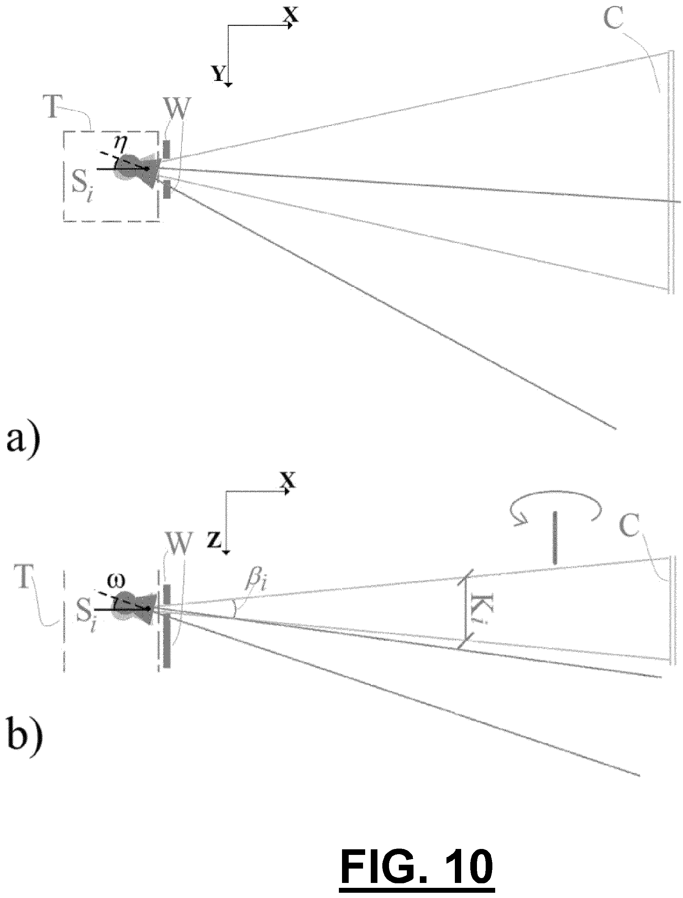

**Figure 10** shows a schematic plan view (a) and a schematic side view (b) of a scanner portion comprising a single source $S_i$, where one can see the angles of rotation, in the plane x-y ($\eta$) and in the plane x-z ($\omega$), by which the sources can be rotated.

Each one of the sources $S_i$ can be rotated in the plane x-y by an angle $\eta$ (**Figure 10a**) and/or in the plane x-z by an angle $\omega$ (**Figure 10b**). Such rotation may occur before the scan process comprising CA acquisition cycles, or it may occur during the scan process. The collimator W can be adjusted as a function of such rotation to have the beam irradiate the desired FOV. Likewise, each one of the sources $S_i$ can be translated in one or more of the three directions x-y-z either before or during the scan process. In this case as well, the apertures of the collimators W may be modified as a function of the translational movements being made. Each one of the N sources $S_i$ may have different angles of rotation $\omega$ and $\eta$, and may be configured with an arbitrary translation along all three directions independently of the other N-1 sources.

In order to produce the 3D image of the map of attenuation coefficients, special reconstruction software is used which is inputted the acquired projections, whether preprocessed or not, and the scan parameters. The reconstruction software implements, in association with or as an alternative to conventional CBCT reconstruction techniques (filtered back-projection - FDK, iterative, AI), an algorithm that builds a weight matrix which makes it possible to linearly combine the contributions from the various sources.

In more detail, for each projection back-projected in the three-dimensional space, such software generates a 3D matrix whose dimensions and number of voxels are equal to those of the reconstructed volume.

Each back-projected projection contributes, therefore, to both the three-dimensional reconstruction of the inspected volume V(x, y, z) and the construction of the weight matrix W(x, y, z), wherein the values 1 or 0 are allocated in each voxel according to the following criterion:

- $W(x_i, y_i, z_i)$ takes value 1 if $V(x_i, y_i, z_i) \neq 0$;
- $W(x_i, y_i, z_i)$ takes value 0 if $V(x_i, y_i, z_i) = 0$.

Therefore, the voxels of such matrix will increment their value by 1 in those positions where said projection gives a non-null contribution to the generation of the 3D image of the examined object.

At the end of the reconstruction, each voxel of the matrix will have a value equal to the number of projections that have contributed in a non-null manner to the value of the voxel having the same coordinates in the reconstructed volume. Those voxels of the weight matrix which have a null value correspond to reconstructed voxels that are external to the FOV, and therefore will not have to give any contribution to the axial combination of the reconstructed volumes related to the sources $S_i$.

To this end, the final values of the voxels of the weight matrix will be suitably renormalized, or, as an alternative, a unitary value will be attributed to null-value voxels.

The volume reconstructed from all sources and acquisition cycles will then be divided, voxel by voxel, by said weight matrix to generate the final 3D image $I(x,y,z) = V(x,y,z)/W(x,y,z)$.

As an alternative, a 3D matrix may be reconstructed for each one of the sources $S_i$, and the total matrix will be obtained by suitably overlapping, in the 3D space, the N matrices derived from the individual sources, appropriately weighted according to the weight matrix.

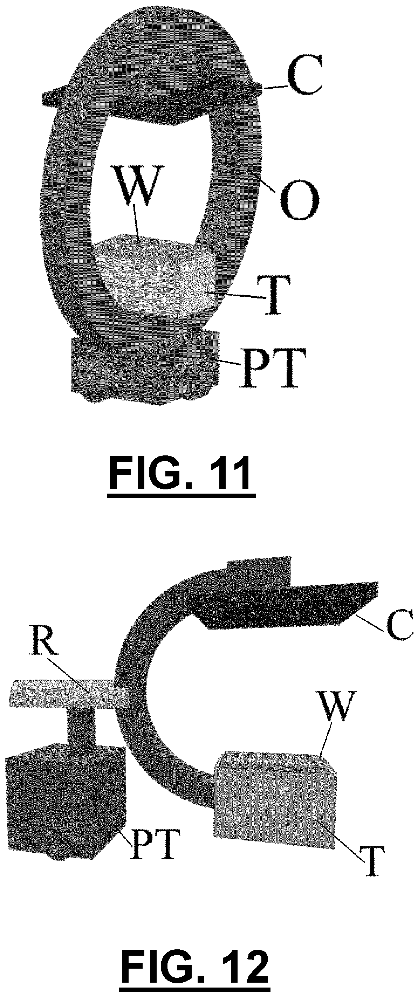

The technology proposed herein can be installed on a dedicated device, even a mobile one, which also comprises, in addition to all essential components, a support providing rotation during the tomographic scan (**Figure 11**, **12**).

**Figure 11** schematically shows an application of the scanner according to the invention, wherein the system of sources (T) and adjustable collimators (W) is mounted on a toroidal support (O) allowing it to rotate about an isocenter, whether integrally or not with the detection system (C). This implementation example also includes a scanner drive system (RT). The support system O is equipped with an internal drive system providing the translations and/or rotations of both C and T, or of T only.

Alternatively, the scanner may be mounted on a C-arm for pre-surgery and post-surgery evaluations (**Figure 12**), i.e. replacing conventional centering CBCT on radiotherapy linear accelerators (LINAC). The system can also be implemented as traditional technology, by using a single source and appropriately adjusting the apertures of the collimation system.

More particularly, **Figure 12** schematically shows an application of the scanner according to the invention, wherein the system of sources (T) and adjustable collimators (W) and the detection system are mounted on a C-arm allowing the former to rotate about an isocenter integrally with the detection system (C). This implementation example also includes a scanner drive system (RT).

The qCBCT technology of the invention makes it possible to remove the limitations of conventional CBCT, caused by excess scattered radiation and by cone artifacts, which are mainly due to the circular orbit of the source. Such improvements have a remarkable effect on image quality, also in relation to the position in the reconstructed field of view, and on the use of the obtained 3D images in clinical practice.

First of all, the reduced scattered X radiation implies a reduction in high-frequency noise, resulting in an improved signal(contrast)-to-noise ratio in the reconstructed images.

This reduces uncertainty in the estimates of Hounsfield Units (HU) and improves feature delineation, resulting in higher measurement precision and repeatability. As is known, the Hounsfield scale is a scale of units of measure which is used for quantifying radiodensity.

In the second place, such reduction in scattered radiation improves the visibility of small lesions or low-contrast inclusions.

Furthermore, reduced scattered radiation results in a considerable reduction in the cupping and shading artifacts that impairs the signal trend in the reconstructed images and that causes low accuracy in quantitative evaluations. The reduction or suppression of such artifacts decreases the need for using post-processing algorithms or technological solutions to restore the quality of the image prior to quantitative analyses. Another consequence of reduced cone artifacts and improved image quality in reconstructed planes distant from the center one (i.e. the plane containing the orbit of the source in CBCT) is an extended usable FOV, which is only limited by the extension of the system of sources and/or of the detector.

The improved image quality provided by qCBCT over traditional CBCT makes it possible to reduce the difference, in terms of image quality and extension of the usable FOV, from Multi-Detector Helical CT (MDCT), which is currently considered the gold standard for quantitative tests, so that the scanner can be employed also for clinical applications where the use of CBCT was previously limited or even impossible, such as:

1) quantitative imaging for radiotherapy planning;
2) detection and characterization of small lesions of the musculoskeletal system;
3) measurement of bone density;
4) ultra-low-dose maxillofacial imaging;
5) pre-surgery and post-surgery evaluations for dental implants;
6) intra-operative and post-operative surgical evaluations;
7) spectral CBCT;
8) automation of image segmentation;
9) extraction of radiomic features;
10) 3D breast imaging; improved visibility of microcalcification in breast-dedicated CT.

Another strong point of the qCBCT configuration is the possibility of using it like traditional CBCT, by suitably presetting the CPU for use of a single source of the assembly and by using a wide-field collimator. The fact that this technology can also be used with the traditional approach can be useful to promote its introduction even in environments where novel technologies are generally viewed with scepticism.

In addition to medical applications, the use of qCBCT may also be beneficial in quality control processes for industrial products. Indeed, the innovative scan orbit introduced by this technology provides more accurate 3D object representation and more exact non-destructive defect and metrological analyses.

The above-described non-limiting examples may be subject to further variations without however departing from the protection scope of the present invention, including all equivalent embodiments known to a person skilled in the art.

The elements and features shown in the various preferred embodiments may be combined together without however departing from the protection scope of the present invention.

From the above description, those skilled in the art will be able to produce the object of the invention without introducing any further details.

**Claims**

**1.** Device for X-ray cone-beam computed tomography, comprising:

- a rotation system (R) for rotating said device around an axis of rotation (D);
- a support system (P) for supporting said device;
- an emission system (T) adapted to emit multiple X-ray cone beams (S);

- an adjustable collimation system (W) adapted to shape said X-ray cone beams, said collimation system being placed in front of said emission system (T);
- a detection system (C) for detecting the X-rays emitted by said emission system (T) and collimated by said collimation system (W),
- said emission system (T) and said collimation system (W) being positioned on a first side of said support (P), and said detection system (C) being positioned on a second side of said support (P), opposite the first side;
- a control system (CPU) adapted to acquire projective views of said X-rays detected by said detection system (C) through one or more acquisition cycles, such that:

- the complete scanning of a volume of interest of an object or an anatomical region (E) placed between said collimation system (W) and said detection system (C) is effected through acquisition of projective images during multiple scan cycles, each cycle including the acquisition of projective views from more than one of said multiple sources (S);
- the X-ray beams emitted by said sources (S) are collimated in such a way as to entirely cover the extension of a field of view (FOV) for each scan cycle;
- the entire tomographic acquisition includes more than one scan cycle, carried out by rotating said rotation system (P) around a volume of interest of said object or anatomical region (E).

2. Device as in claim 1, further comprising a reconstruction system (CPU$_R$) adapted to use said projective views acquired by said control system (CPU) and reconstruct a tomographic volume.

3. Device as in claim 1 or 2, wherein said detection system (C) is not rotatably integral with the system that comprises the plurality of sources.

4. Device as in any one of the preceding claims, wherein said multiple X-ray sources (S) are arranged on a segment that is parallel to the axis of rotation, and the central axis of the emitted beams is contained in a plane perpendicular to such axis, or said multiple X-ray sources (S) are not coplanar to one another or arranged on the same segment, or said multiple X-ray sources (S) are arranged on an arc of circumference and said X-ray beams are rotated.

5. Device as in any one of the preceding claims, wherein said X-ray sources are controlled by said control system during the rotation in such a way as to create a quasi-sinusoidal scan orbit on a semi-period, or a sawtooth (raster) scan orbit.

6. Device as in any one of the preceding claims, wherein said control system (CPU) executes said one or more scan cycles by acquiring projective views from a subset of said sources that constitute said system of X-photon sources.

7. Device as in any one of the preceding claims, wherein said detector (C) is sized to detect one or some of said multiple X-ray beams and is adapted to translate vertically, said control system (CPU) executing said vertical translation of the detector in coordination with the emission of each one of said sources (S), or said detector (C) comprises a plurality of detectors (Ci) having such transverse dimensions that each one of them allows the detection of a corresponding source (Si) of X-ray beams or portions thereof.

8. Device as in any one of the preceding claims, wherein each one of said sources (Si) is rotated, and said collimator (W) is adjusted as a function of such rotation under control of said control system (CPU).

9. Device as in any one of the preceding claims, wherein said control unit (CPU) controls whether said device must operate in step-and-shoot mode or in continuous mode or in pulsed mode.

10. Device as in any one of the preceding claims, wherein said collimator (W) is made of a material with a high atomic number or an alloy of materials having a high attenuation coefficient.

11. Device as in any one of the preceding claims, wherein said emission system (T) comprises multiple tube-shaped X-ray sources, or a multi-spot source, i.e. a single source having multiple focal points or spots from which said X-rays are generated, or said emission system (T) is an assembly of multiple X-ray sources, multi-spot X-ray sources, or single-spot X-ray sources.

12. Device as in claim 2, wherein said reconstruction system (CPU$_R$) implements an algorithm which constructs a weight matrix that makes it possible to linearly combine said projective views, for each projective view, back-projected in a three-dimensional space, to generate a 3D matrix whose dimensions and number of voxels are equal to those of said reconstructed tomographic volume.

13. Device as in any one of the preceding claims, wherein said collimation system (W) comprises slots (F) through which said X-ray cone beams can pass, said slots (F) spatially limiting the aperture of said X-ray cone beams and determining said shape, the size of said slots being adjustable by means of actuators controlled by said control system (CPU).

14. Device as in any one of the preceding claims, wherein said device is comprised in a C-arm or a centering system of a radiotherapy linear accelerator (LINAC).

**FIG. 1**

# FIG. 2

# FIG. 4

a)

b)

# FIG. 3

**FIG. 5**

**FIG. 6**

**FIG. 7**

## FIG. 8

## FIG. 9

16

a)

b)

# FIG. 10

**FIG. 11**

**FIG. 12**

Europäisches
Patentamt
European
Patent Office
Office européen
des brevets

## EUROPEAN SEARCH REPORT

**Application Number**

EP 25 18 4712

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BECKER AMY E ET AL: "Cone beam CT multisource configurations: evaluating image quality, scatter, and dose using phantom imaging and Monte Carlo simulations", PHYSICS IN MEDICINE AND BIOLOGY , vol. 65, no. 23 27 November 2020 (2020-11-27), page 235032, XP093250626, Bristol GB ISSN: 0031-9155, DOI: 10.1088/1361-6560/abc306 Retrieved from the Internet: URL:https://iopscience.iop.org/article/10.1088/1361-6560/abc306 * sections 2.1-2.2, 3.1.4, 4.2; figure 1d * ----- | 1-14 | INV. G01N23/046 A61B6/03 |
| X | WO 2023/238108 A1 (ISTITUTO NAZ FISICA NUCLEARE [IT]) 14 December 2023 (2023-12-14) * page 11, line 15 - page 11, line 22; figures 1-5 * * page 15, line 16 - page 16, line 2 * * page 18, line 4 - page 18, line 5 * ----- | 1-14 | **TECHNICAL FIELDS SEARCHED (IPC)** G01N A61B |

The present search report has been drawn up for all claims

| Place of search | Date of completion of the search | Examiner |
|---|---|---|
| The Hague | 14 October 2025 | Stavroulakis, E |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P04C01)

**EP 4 679 066 A1**

## ANNEX TO THE EUROPEAN SEARCH REPORT
## ON EUROPEAN PATENT APPLICATION NO.

EP 25 18 4712

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2025

| Patent document cited in search report | Publication date | Patent family member(s) | Publication date |
|---|---|---|---|
| WO 2023238108 A1 | 14-12-2023 | EP 4536087 A1<br>WO 2023238108 A1 | 16-04-2025<br>14-12-2023 |

EPO FORM P0459

For more details about this annex : see Official Journal of the European Patent Office, No. 12/82